# EUROPÄISCHE PATENTSCHRIFT

(11) **EP 0 370 391 B1**
(45) Veröffentlichungstag und Bekanntmachung des Hinweises auf die Patenterteilung: **02.11.1994**
(21) Anmeldenummer: 89121242.5
(22) Anmeldetag: 16.11.1989
(51) Int. Cl.: C07D 239/30

(54) **Verfahren zur Herstellung von 4,5-Dichlor-6-ethylpyrimidin**
Process for the preparation of 4,5-dichloro-6-ethyl pyrimidine
Procédé de préparation de 4,5-dichloro-6-éthylpyrimidine

(30) Priorität: 21.11.1988 CH 4306/88
(43) Veröffentlichungstag der Anmeldung: 30.05.1990
(73) Patentinhaber: LONZA AG, CH-3945 Gampel/Wallis (CH)
(72) Erfinder: Mills, Lester, Dr., Naters (Kanton Wallis) (CH); Previdoli, Felix, Dr., Brig (Kanton Wallis) (CH)
(74) Vertreter: Weinhold, Peter, Dr.

(56) Entgegenhaltungen:
- GB-A- 1 585 950
- RECUEIL DES TRAVAUX CHIMIQUES DES PAYS-BAS, Band 97, Nr. 11, November 1965,Seiten 288-292, Den Haag, NL; C.A.H. RASMUSSEN et al.: "Aspects of theamination of 4-t-butyl-5-halogenopyrimidines by potassium amide in liquidammonia"
- Beilstein "Handbuch der organischen Chemie", Band 3, Seite 662, "Oxo-carbonsäuren, Substitutionsprodukte"

## Beschreibung

4,5-Dichlor-6-ethylpyrimidin ist ein wertvolles Zwischenprodukt zur Herstellung von 4-Amino-5-chlor-6-ethylpyrimidinen, die insectizide, acarizide und fungizide Eigenschaften aufweisen (EP-A 0 264 217). Die bisher bekannten Verfahren zur Herstellung von 4,5-Dichlor-6-alkylpyrimidinen gehen von den entsprechenden 4-Hydroxy-6-alkylpyrimidinen aus, welche zunächst mit elementarem Chlor oder einem chlorübertragenden Mittel wie N-Chlorsuccinimid in die 5-Chlor-4-hydroxy-6-alkylpyrimidine übergeführt werden (JP-A 222070/83). Letztera werden dann mit Phosphorylchlorid oder einem anderen anorganischen Säurechlorid in die 4,5-Dichlor-Verbindungen umgewandelt. Dieses Verfahren ist zwar an sich mit guter Ausbeute durchführbar und auch für den industriellen Massstab geeignet, nachteilig ist jedoch, dass die erforderlichen 4-Hydroxy-6-alkylpyrimidine nur schlecht zugänglich sind. Ihre Synthese geht üblicherweise von dem entsprechenden 2-Thio-6-alkyluracil aus, welches mit Raney-Nickel entschwefelt wird. Die 2-Thio-6-alkyluracile sind zwar aus den entsprechenden 3-Oxocarbonsäureestern und Thioharnstoff leicht herzustellen (H.M.Foster und H.R.Snyder, Org. Synth., Coll. Vol. IV, 638), die Entschwefelung mit Raney-Nickel ist jedoch für ein technisches Verfahren nicht praktikabel.

In der GB-A-1 585 950 wird die Herstellung von Alkylpyrimidinonen durch Umsetzung von β-Ketoestern mit Amidinderivaten unter dem Einfluß von Alkalialkoholaten beschrieben. Diese Pyrimidinone müssen ebenfalls zunächst mit einem chlorübertragenden Mittel in 5-Chlor-4-hydroxy-6-alkylpyrimidine überführt werden, bevor sie mit Phosphorylchlorid zum gewünschten 4,5-Dichlor-6-alkylpyrimidin umgesetzt werden können.

Rasmussen, C.A.H. und van der Plas, H.C. (Rel. Trav. Chim. Pays-Bas 97, 288-292; 1978) beschreiben u.a. die Herstellung von 4-t-Butyl-5,6-dichlorpyrimidin. Hierzu wird durch Umsetzung eines Formamidiniumsalzes mit 4,4-Dimethyl-3-oxovalerat zunächst das 4-t-Butyl-6-pyrimidinon hergestellt, das anschliessend zuerst mit Chlor in 4-t-Butyl-5-chlor-6-pyrimidinon überführt wird und dann mit Phosphorylchlorid zum gewünschten Dichlorpyrimidin umgesetzt wird.

Aufgabe der Erfindung war daher, ein Verfahren zur Herstellung von 4,5-Dichlor-6-ethylpyrimidin zur Verfügung zu stellen, das auch im technischen Massstab wirtschaftlich durchgeführt werden kann.

Erfindungsgemäss wird diese Aufgabe dadurch gelöst, dass ein 2-Chlor-3-oxopentansäureester der allgemeinen Formel 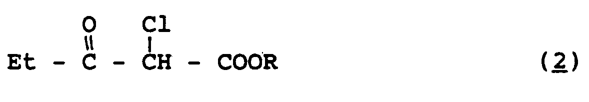
zunächst in einem polaren protischen Lösungsmittel in Gegenwart einer Base mit einem Formamidiniumsalz der allgemeinen Formel 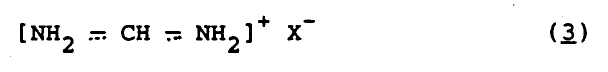 zu 5-Chlor-6-ethyl-4-hydroxypyrimidin (4) kondensiert und dieses anschliessend in an sich bekannter Weise mit Phosphorylchlorid in die Zielverbindung übergeführt wird.
Als 2-Chlor-3-oxopentansäureester findet zweckmässig ein niedriger Alkylester Verwendung, wobei R eine Alkylgruppe mit 1 bis 4 C-Atomen ist, also ein Methyl-, Ethyl-, Propyl-, Isopropyl-, Butyl-, Isobutyl-, sec-Butyl- oder tert-Butylester, vorzugsweise der Methyl- oder Ethylester, besonders bevorzugt der Methylester. Diese Ester sind aus den entsprechenden 3-Oxopentansäureestern durch Chlorierung, vorzugsweise mit Sulfurylchlorid, leicht erhältlich. Die Herstellung der 3-Oxopentansäureester ist aus der CH-PS 655 301 bekannt. In einer bevorzugten Ausführungsform des Verfahrens wird der 2-Chlor-3-oxopentansäureester mit Sulfurylchlorid hergestellt und in der nach Abtreiben der gasförmigen Reaktionsprodukte und Abdestillieren von überschüssigem Sulfurylchlorid vorliegenden rohen Form ohne Reinigung eingesetzt.

Als Formamidiniumsalz wird zweckmässig ein Salz des Formamidins mit einer starken anorganischen Säure wie Salzsäure oder Schwefelsäure oder einer niedrigen aliphatischen Carbonsäure d.h. einer solchen von 1-4 Kohlenstoffatomen wie Essigsäure eingesetzt. Bevorzugte Formamidiniumsalze sind Formamidiniumchlorid und Formamidiniumacetat. Als polares protisches Lösungsmittel wird zweckmässig ein niedriger Alkohol, vorzugsweise der dem eingesetzten 2-Chlor-3-oxopentansäureester entsprechende, besonders bevorzugt also Ethanol oder insbesondere Methanol verwendet.

Als Base eignet sich jede Base, die keine unerwünschten Nebenreaktionen hervorruft und im verwendeten Lösungsmittel hinreichend löslich ist. Vorzugsweise werden Alkalialkoholate von Lithium, Natrium oder Kalium mit niedrigen Alkoholen, insbesondere mit 1 bis 4 C-Atomen, eingesetzt. Besonders bevorzugt sind die Natriumalkoholate, vor allem jeweils das von dem auch als Lösungsmittel verwendeten Alkohol abgeleitete. Zur Unterdrückung von Nebenreaktionen hat es sich als besonders vorteilhaft erwiesen, das Alkalialkoholat nach Massgabe des Reaktionsfortschritts langsam zu der vorgelegten Mischung des 2-Chlor-3-oxopentansäureesters mit dem Formamidiniumsalz zuzudosieren.
Eine weitere bevorzugte Ausführungsform der Reaktion verwendet Kaliumcarbonat als Base und Methanol als Lösungsmittel. Hierbei sorgt die begrenzte Löslichkeit des Kaliumcarbonats für eine niedrige Basenkonzentration in Reaktionsgemisch, so dass die Gesamtmenge Kaliumcarbonat auf einmal zugegeben werden kann und sich dann allmählich auflöst.

Die Reaktion zwischen dem 2-Chlor-3-oxopentansäureester und dem Formamidiniumsalz wird vorteilhaft bei relativ niedriger Temperatur, vorzugsweise bei 0 bis 30°C durchgeführt. Vorzugsweise wird dabei das Formamidiniumsalz in leichtem Ueberschuss eingesetzt, nämlich bis zum 1,5-fachen der theoretisch erforderlichen Menge.
Für 1 Mol 2-Chlor-3-pentansäureester werden zweckmässig 2 Aequivalente Base oder vorzugsweise eine geringfügig grössere Menge eingesetzt.
Nach Beendigung der Reaktion zu 5-Chlor-6-ethyl-4-hydroxypyrimidin kann dieses nach einer der üblichen Methoden isoliert, gegebenenfalls gereinigt, und anschliessend in an sich bekannter Weise mit Phosphorylchlorid in das 4,5-Dichlor-6-ethyl-pyrimidin übergeführt werden. In einer bevorzugten Ausführungsform wird jedoch die 4-Hydroxy-Verbindung nicht isoliert, sondern das Reaktionsgemisch wird zunächst, zweckmässig durch Einleiten von gasförmigem Chlorwasserstoff bis zur deutlich sauren Reaktion (pH ≦ 3), angesäuert. Anschliessend wird, vorzugsweise unter vermindertem Druck, das Lösungsmittel, zweckmässig Methanol oder Ethanol, weitgehend abdestilliert. Nach Zugabe eines höhersiedenden inerten Lösungsmittels, das als Schlepper wirkt, wird der restliche Alkohol sowie gegebenenfalls noch vorhandenes Wasser und/oder Essigsäure abdestilliert.
"Inert" bedeutet in diesem Zusammenhang lediglich, dass das höhersiedende Lösungsmittel bei der abschliessenden Umsetzung mit Phosphorylchlorid keine störenden Reaktionen eingeht.
Als inerte höhersiedende Lösungsmittel eignen sich beispielsweise alkylierte aromatische Kohlenwasserstoffe wie Toluol, Xylol oder Ethylbenzol, vorzugsweise wird Toluol eingesetzt.

Eine andere Verwendung des erfindungsgemäss hergestellten 5-Chlor-6-ethyl-4-hydroxypyrimidins besteht darin, dieses in Analogie zu bekannten Verfahren mit Wasserstoff an einem Palladiumkatalysator hydrogenolytisch zu dehalogenieren. Damit ist auch das 6-Ethyl-4-hydroxypyrimidin unter Vermeidung der eingangs genannten Nachteile gut zugänglich.

Die nachfolgenden Beispiele verdeutlichen die Ausführung der Erfindung.

### Beispiel 1

### 5-Chlor-6-ethyl-4-hydroxypyrimidin

Zu einer Lösung von 8,6 g (0,049 mol) 2-Chlor-3-oxopentansäuremethylester (Gehalt 94%) und 6,04 g (0,075 mol) Formamidiniumchlorid in 10 ml Methanol wurden bei 20 bis 22°C unter Rühren innerhalb von 2 Stunden 21,6 g Natriummethylatlösung (30%ig in Methanol, 0,12 mol) zudosiert. Nach weiteren 3 Stunden Rühren wurde mit conc. Salzsäure neutralisiert (pH≈5) und am Rotationsverdampfer zur Trockne eingedampft. Der Rückstand wurde mit 20 ml Wasser aufgenommen und dreimal mit Ethylacetat ausgeschüttelt. Die vereinigten organischen Phasen wurden über Natriumsulfat getrocknet, das Lösungsmittel abdestilliert und der feste Rückstand bei 50°C und 100 Torr getrocknet.
- Ausbeute:: 7,42 g (93%)
- Schmelzpunkt:: 144 bis 145°C
- ¹H-NMR:: (CDCl₃, 300 MHz) δ = 1,30 (t, 3H), 2,85 (q, 2H), 8,20 (s, 1H), 13,05 (br.s, 1H)

### Beispiel 2

Ein Gemisch von 5,0 g (0,030 mol) 2-Chlor-3-oxopentansäuremethylester (Gehalt 98,6%), 3,14 g (0,039 mol) Formamidiniumchlorid, 9,1 g (0,066 mol) Kaliumcarbonat und 25 g Methanol wurde bei 10°C 5,5 Stunden gerührt. Anschliessend wurde das Gemisch mit conc. Salzsäure auf pH≈5 angesäuert und wie in Beispiel 1 beschrieben aufgearbeitet.
- Ausbeute:: 3,8 g (78%)

### Beispiel 3

Zu einer Lösung von 5,35 g (0,030 mol) 2-Chlor-3-oxopentansäuremethylester (Gehalt 93,5%) und 2,93 g (0,036 mol) Formamidiniumchlorid in 5,1 g Ethanol wurden innerhalb von 3 Stunden bei 10°C 19,15 g Natriumethylatlösung (22,5%ig in Ethanol, 0,063 mol) unter Rühren zudosiert. Die Aufarbeitung erfolgte wie im Beispiel 1 beschrieben.
- Ausbeute:: 4,74 g (95%)

### Beispiel 4

Zu einer Lösung von 5,35 g (0,030 mol) 2-Chlor-3-oxopentansäuremethylester (Gehalt 93,5%) und 2,92 g (0,036 mol) Formamidiniumchlorid in 5,1 g Methanol wurden 3,08 g (0,030 mol) Triethylamin (als 30%ige Lösung in Methanol) innerhalb von 4,5 Stunden bei 10°C unter Rühren zudosiert. Die Aufarbeitung erfolgte wie in Beispiel 1 beschrieben.
- Ausbeute:: 3,14 g (64%)

### Beispiel 5

Zu einer Lösung von 6,7 g (0,040 mol) 2-Chlor-3-oxopentansäuremethylester (Gehalt 98%) und 4,79 g (0,046 mol) Formamidiniumacetat in 5 g Methanol wurden innerhalb von 30 Minuten bei 12°C unter Rühren 14,4 g Natriummethylatlösung (30%ig in Methanol, 0,08 mol) zudosiert. Die Aufarbeitung erfolgte wie im Beispiel 1 beschrieben.
- Ausbeute:: 5,41 g (82%)

### Beispiel 6

Zu einer Lösung von 5,42 g (0,030 mol) 2-Chlor-3-oxopentansäuremethylester (Gehalt 92,2%) und 3,75 g (0,018 mol) Formamidiniumsulfat in 5 g Methanol wurden innerhalb von 30 Minuten bei 10°C unter Rühren 11,4 g Natriummethylatlösung (30%ig in Methanol, 0,063 mol) zudosiert. Die Aufarbeitung erfolgte wie im Beispiel 1 beschrieben.
- Ausbeute:: 2,06 g (42%)

### Beispiel 7

### 4,5-Dichlor-6-ethylpyrimidin

Ein Gemisch von 3,19 g (0,020 mol) 5-Chlor-4-ethyl-6-hydroxypyrimidin, 15,33 g (0,10 mol) Phosphorylchlorid und 45 g Toluol wurde auf 100°C erhitzt und bei dieser Temperatur 80 Minuten gerührt. Das überschüssige Phosphorylchlorid und das Toluol wurden abdestilliert und der Rückstand mit 30 ml Dichlormethan und 20 ml Wasser versetzt. Das Gemisch wurde nach 30 Minuten am Rückfluss gekocht, mit 3 x 30 ml Dichlormethan extrahiert und über Natriumsulfat getrocknet. Die Dichlormethanlösung wurde eingedampft und der Rückstand bei 125°C/12 Torr im Kugelrohr-Apparat destilliert.
- Ausbeute:: 2,77 g (77%)
- Siedepunkt:: 89 bis 94°C/12 Torr
- ¹H-NMR: (CDCl₃, 300 MHz) δ = 1,36 (t, 3H), 3,00 (q, 2H), 8,79 (s, 1H)

### Beispiel 8

### 4,5-Dichlor-6-ethylpyrimidin (Eintopfverfahren)

Zu 53,6 g (0,40 mol) 3-Oxopentansäuremethylester wurden bei 12°C innerhalb von 25 Minuten 56,8 g (0,416 mol) Sulfurylchlorid unter Rühren zugegeben. Die entstehenden Gase (HCl, SO₂) wurden in Wasser absorbiert. Anschliessend wurde das überschüssige Sulfurylchlorid zusammen mit den restlichen gasförmigen Produkten im Vakuum entfernt und der Rückstand mit 56,2 g (0,54 mol) Formamidiniumacetat und 50 g Methanol gemischt. Bei 12 bis 16°C wurden 172 g Natriummethylatlösung (30%ig in Methanol, 0,96 mol) innerhalb von 45 Minuten zudosiert. Es wurde noch weitere 2,5 Stunden bei 12°C gerührt und dann Chlorwasserstoffgas eingeleitet, bis ein pH-Wert von ca. 2 erreicht war (ca. 20 Minuten). Bei 90°C wurde zunächst die Hauptmenge Methanol abdestilliert, dann 500 g Toluol zugesetzt und die Temperatur langsam erhöht, bis nur noch Toluol überdestillierte (Kopftemperatur ca. 114°C). Nach Abkühlen auf 80°C wurden 291 g (1,89 mol) Phosphorylchlorid innerhalb von 30 Minuten zugegeben, wobei der Temperaturanstieg auf weniger als 5° begrenzt wurde. Anschliessend wurde das Gemisch noch 4,5 Stunden bei 80°C gerührt, das überschüssige Phosphorylchlorid mit dem Toluol weitgehend abdestilliert und der viskose Rückstand mit Toluol verdünnt. Das so erhaltene Gemisch wurde auf 20°C gekühlt und so vorsichtig mit 400 ml Wasser versetzt, dass die Temperatur nicht über 40°C anstieg. Die dunkel gefärbte organische Phase wurde abgetrennt, mit Natriumhydrogencarbonatlösung gewaschen, über Natriumsulfat getrocknet und das Toluol abdestilliert. Der Rückstand (ca. 52 g) wurde im Wasserstrahlvakuum fraktioniert.
- Ausbeute:: 46,4 g (65,5%, bezogen auf 3-Oxopentansäuremethylester)

### Beispiel 9

Zu 28,8 g (0,162 mol) 2-Chlor-3-oxopentansäuremethylester (Gehalt 92,2%) und 22,5 g (0,21 mol) Formamidiniumacetat in 20 g Methanol wurden innerhalb von 20 Minuten bei 11°C unter Rühren 69 g Natriummethylatlösung (30%ig in Methanol, 0,38 mol) zudosiert. Nach weiteren 3 Stunden Rühren bei 11°C wurden 20 g (0,55 mol) Chlorwasserstoffgas eingeleitet, wobei ein pH von ca. 3 erreicht wurde. Das Methanol wurde unter vermindertem Druck abdestilliert (40 bis 45°C, 250 Torr), 180 ml Toluol wurden zugegeben und das restliche Methanol sowie die Nebenprodukte Wasser und Essigsäure vollends abdestilliert. Das zurückbleibende Gemisch wurde auf 80°C gebracht und mit 115 g (0,75 mol) Phosphorylchlorid versetzt. Nach 4 Stunden Rühren bei 80 bis 90°C wurde das überschüssige Phosphorylchlorid abdestilliert (42 bis 45°C, 250 Torr). Der Rückstand wurde mit 100 ml Toluol verdünnt und anschliessend langsam bei 11 bis 20°C mit 140 ml Wasser versetzt. Die organische Phase wurde abgetrennt, mit je 20 g gesättigter Natriumhydrogencarbonatlösung und Wasser gewaschen und im Vakuum (12 Torr) fraktioniert.
- Ausbeute:: 22,7 g (95%ig) (76%, bezogen auf 2-Chlor-3-oxopentansäuremethylester)

### Beispiel 10 (Mengen normiert auf 1000 g Produktausbeute)

Zu 1095 g 3-Oxopentansäuremethylester wurden bei 12°C unter Rühren innerhalb von 30 Minuten 1148 g Sulfurylchlorid zugetropft. Nach einer weiteren Stunde Reaktionsdauer wurde das überschüssige Sulfurylchlorid im Wasserstrahlvakuum abgezogen, wobei sich das Reaktionsgemisch auf ca. 5°C abkühlte und dann wieder allmählich auf ca. 10°C erwärmte. Anschliessend wurde zuerst eine Lösung von 1165 g Formamidiniumacetat in 1036 g Methanol zugegeben und danach eine Lösung von 1071 g Natriummethylat in 2500 g Methanol zugetropft, wobei die Temperatur auf 16°C anstieg. Nach ca. 2,5 Stunden wurde das Reaktionsgemisch durch Einleiten von ca. 800 g HCl-Gas innerhalb von ca. 30 Minuten auf einen scheinbaren pH von 3 gebracht. Die Temperatur stieg dabei auf 21°C. Anschliessend wurde zunächst bei 40 bis 50°C und 500 Torr das Methanol mit der Hauptmenge der Nebenprodukte Wasser und Essigsäure abdestilliert. Der Rest des Methanol/Wasser/Essigsäure-Gemisches wurde nach entsprechendem Erhitzen unter kontinuierlicher Zugabe von 4520 g Toluol zusammen mit diesem azeotrop abdestilliert, bis im Destillat keine Essigsäure mehr nachweisbar war. Nach Abkühlen auf 88°C wurde innerhalb von 30 Minuten ein Gemisch von 6037 g Phosphorylchlorid und 496 g Toluol zugetropft. Nach 3 Stunden Reaktionsdauer wurde der Überschuss an Phosphorylchlorid bei 60°C und einem Druck von 200 Torr, der allmählich auf 50 Torr abgesenkt wurde, abdestilliert. Der Rückstand wurde mit 5427 g Toluol verdünnt, auf 20°C abgekühlt und vorsichtig mit 8,29 l Wasser versetzt. Nach 20 Minuten Rühren wurde die wässrige Phase abgetrennt und die organische Phase einmal mit 2176 g gesättigter Natriumhydrogencarbonatlösung gewaschen. Das Toluol wurde bei 60°C unter vermindertem Druck abdestilliert, bis ein Vakuum von 12 Torr erreicht war. Schliesslich wurde der Rückstand bei 12 Torr destilliert (Siedebereich: 89 bis 94°C).

## Patentansprüche

1. Verfahren zur Herstellung von 4,5-Dichlor-6-ethylpyrimidin 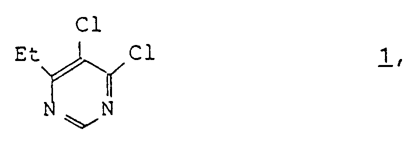 dadurch gekennzeichnet, dass ein 2-Chlor-3-oxopentansäureester der allgemeinen Formel 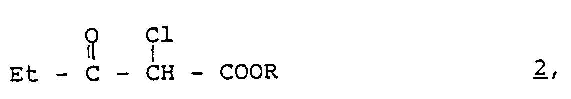 wobei R eine Alkylgruppe mit 1 bis 4 C-Atomen bedeutet, mit einem Formamidiniumsalz 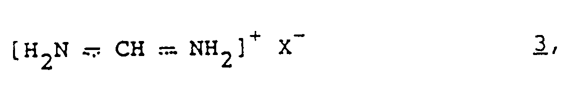 wobei X⁻ das Anion einer starken anorganischen Säure oder einer niedrigen aliphatischen Carbonsäure bedeutet, in Gegenwart einer Base in einem polaren protischen Lösungsmittel zu 5-Chlor-6-ethyl-4-hydroxypyrimidin 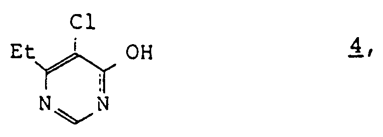 kondensiert und dieses mit Phosphorylchlorid zum gewünschten Produkt 1 umgesetzt wird.

2. Verfahren nach Patentanspruch 1, dadurch gekennzeichnet, dass als Base ein Alkalialkoholat der allgemeinen Formel M O-R′, worin M Lithium, Natrium oder Kalium und R′ eine Alkylgruppe mit 1 bis 4 C-Atomen ist, und als Lösungsmittel der entsprechende Alkohol eingesetzt wird.

3. Verfahren nach Patentanspruch 2, dadurch gekennzeichnet, dass als Alkalialkoholat Natriummethylat oder Natriumethylat eingesetzt wird.

4. Verfahren nach mindestens einem der Patentansprüche 2-3, dadurch gekennzeichnet, dass die Alkalialkoholat-Lösung langsam zu einer vorgelegten Mischung, die den 2-Chlor-3-oxopentansäureester und das Formamidiniumsalz enthält, zudosiert wird.

5. Verfahren nach Patentanspruch 1, dadurch gekennzeichnet, dass als Base Kaliumcarbonat und als Lösungsmittel Methanol eingesetzt wird.

6. Verfahren nach mindestens einem der Patentansprüche 1-5, dadurch gekennzeichnet, dass als 2-Chlor-3-oxopentansäureester der Methyl- oder Ethylester eingesetzt wird.

7. Verfahren nach mindestens einem der Patentansprüche 1-6, dadurch gekennzeichnet, dass als Formamidiniumsalz das Acetat oder das Chlorid eingesetzt wird.

8. Verfahren nach mindestens einem der Patentanspruche 3-7, dadurch gekennzeichnet, dass das Reaktionsgemisch nach Bildung des 5-Chlor-6-ethyl-4-hydroxypyrimidins mit Chlorwasserstoffgas angesäuert, das Methanol oder Ethanol unter Zusatz eines höhersiedenden inerten Lösungsmittels vollständig abdestilliert und das zurückbleibende Gemisch ohne weitere Aufarbeitung mit Phosphorylchlorid umgesetzt wird.

9. Verfahren nach Patentanspruch 8, dadurch gekennzeichnet, dass als höhersiedendes inertes Lösungsmittel Toluol eingesetzt wird.

10. Verfahren nach mindestens einem der Patentansprüche 1-9, dadurch gekennzeichnet, dass als 2-Chlor-3-oxopentansäureester direkt das durch Umsetzung des entsprechenden 3-Oxopentansäureesters mit Sulfurylchlorid erhaltene Rohprodukt eingesetzt wird.

## Claims

1. A process for the preparation of 4,5-dichloro-6-ethyl pyrimidine: 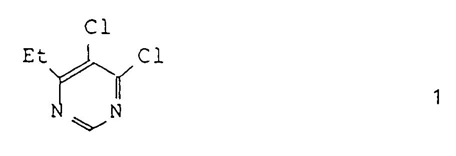 characterized in that a 2-chloro-3-oxopentanoic acid ester of the general formula: 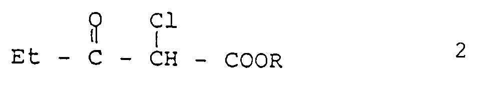 wherein R is an alkyl group having 1 to 4 carbon atoms; is condensed with a formamidinium salt: 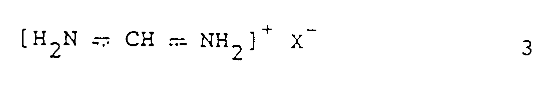 wherein X⁻ is the anion of a strong inorganic acid or of a lower aliphatic carboxylic acid; in the presence of a base and in a polar protic solvent to 5-chloro-6-ethyl-4-hydroxy pyrimidine: 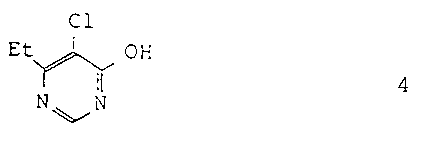 which is then reacted with phosphoryl chloride to the desired product 1.

2. The process according to Claim 1, characterized by using as a base an alkali alcoholate of the general formula M O-R' wherein M is lithium, sodium or potassium, and R' is an alkyl group having 1 to 4 carbon atoms, and as a solvent the respective alcohol.

3. The process according to Claim 2, characterized by using as an alkali alcoholate sodium methylate or sodium ethylate.

4. The process according to at least one of Claims 2 and 3, characterized in that the solution of alkali alcoholate is slowly metered into a mixture containing the 2-chloro-3-oxopentanoic acid ester and the formamidinium salt.

5. The process according to Claim 1, characterized by using potassium carbonate as a base and methanol as a solvent.

6. The process according to at least one of Claims 1 to 5, characterized by using as the 2-chloro-3-oxopentanoic acid ester the methyl or ethyl ester.

7. The process according to at least one of Claims 1 to 6, characterized by using as the formamidinium salt the acetate or the chloride.

8. The process according to at least one of Claims 3 to 7, characterized in that, after formation of the 5-chloro-6-ethyl-4-hydroxy pyrimidine, the reaction mixture is acidified with hydrogen chloride gas, methanol or ethanol are distilled off completely under addition of a higher-boiling inert solvent, and the remaining mixture, without further processing, is reacted with phosphoryl chloride.

9. The process according to Claim 8, characterized by using toluene as a higher-boiling inert solvent.

10. The process according to at least one of Claims 1 to 9, characterized by using as a 2-chloro-3-oxopentanoic acid ester directly the crude product obtained by reaction of the respective 3-oxopentanoic acid ester with sulfuryl chloride.

## Revendications

1. Procédé pour la préparation de 4,5-dichloro-6-éthylpyrimidine 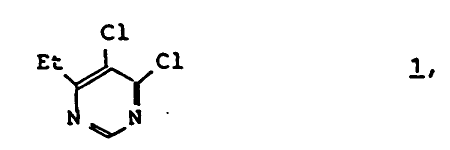 caractérisée en ce que l'on condense un ester de l'acide 2-chloro-3-oxopentanique de la formule générale 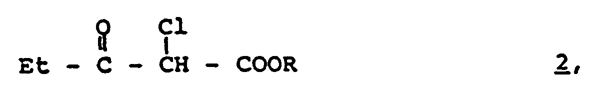 dans laquelle R signifie un groupe alkyle avec 1 à 4 atomes C, avec un sel de formamidinium 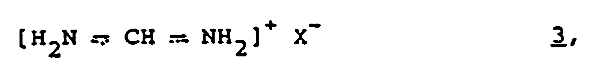 où X⁻ signifie l'anion d'un acide minéral fort ou d'un acide carboxylique aliphatique inférieur en présence d'une base dans un solvant polaire protique en 5-chloro-6-éthyl-4-hydroxypyrimidine 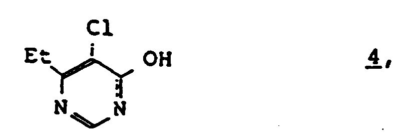 et que l'on met celle-ci en réaction avec le chlorure de phosphoryle pour obtenir le produit 1 souhaité.

2. Procédé selon la revendication 1, caractérisé en ce qu'en tant que base, on met en oeuvre un alcoolat alcalin de formule générale M O-R', dans laquelle M est le lithium, le sodium ou le potassium, et R' est un groupe alkyle avec 1 à 4 atomes C et en tant que solvant, l'alcool correspondant.

3. Procédé selon la revendication 2, caractérisé en ce qu'en tant qu'alcoolat alcalin, on met en oeuvre le méthylate de sodium ou l'éthylate de sodium.

4. Procédé selon au moins l'une des revendications 2-3, caractérisé en ce que l'on ajoute en dosant lentement la solution d'alcoolat alcalin à un mélange introduit qui contient l'ester de l'acide 2-chloro-3-oxopentanique et le sel de formamidinium.

5. Procédé selon la revendication 1, caractérisé en ce qu'en tant que base, on met en oeuvre du carbonate de potassium et en tant que solvant du méthanol.

6. Procédé selon au moins l'une des revendications 1-5, caractérisé en ce qu'en tant qu'ester de l'acide 2-chloro-3-oxopentanique, on met en oeuvre l'ester méthylique ou éthylique.

7. Procédé selon au moins l'une des revendications 1-6, caractérisé en ce qu'en tant que sel de formamidinium, on met en oeuvre l'acétate ou le chlorure.

8. Procédé selon au moins l'une des revendications 3-7, caractérisé en ce que le mélange réactionnel après formation de la 5-chloro-6-éthyl-4-hydroxypyrimidine est acidifié avec un gaz chlorhydrique, le méthanol ou l'éthanol en ajoutant un solvant inerte à point d'ébullition élevé chassé complètement par distillation et l'on met en réaction le mélange restant sans traitement supplémentaire avec le chlorure de phosphoryle.

9. Procédé selon la revendication 8, caractérisé en ce qu'en tant que solvant inerte à point de fusion élevé, on met en oeuvre du toluol.

10. Procédé selon au moins l'une des revendications 1-9, caractérisé en ce qu'en tant qu'ester de l'acide 2-chloro-3-oxopentanique, on met en oeuvre directement le produit brut obtenu par réaction de l'ester de l'acide 3-oxopentanique correspondant avec du chlorure de sulfuryle.
